# EUROPEAN PATENT SPECIFICATION

(11) **EP 0 583 319 B1**
(45) Date of publication and mention of the grant of the patent: **12.07.1995**
(21) Application number: 92909757.4
(22) Date of filing: 30.04.1992
(51) Int. Cl.: D21C 9/08, C12P 7/62

(54) **LIPASE-CATALYZED ESTER HYDROLYSIS**
LIPASE-KATALYSIERTE ESTERHYDROLYSE
HYDROLYSE D'UN ESTER CATALYSEE PAR UNE LIPASE

(30) Priority: 01.05.1991 EP 91610037
(43) Date of publication of application: 23.02.1994
(73) Proprietor: NOVO NORDISK A/S, 2880 Bagsvaerd (DK); NIPPON PAPER INDUSTRIES CO., LTD., Kita-ku, Tokyo 114 (JP)
(72) Inventor: FUJITA, Yuko, Higashiizumi 3-7-23-102, Tokyo 201 (JP); AWAJI, Haruo, Hasunuma 805-2, Saitama-ken 330 (JP); SHIMOTO, Hidesato, Kouyadai 1-14-24, Chiba-ken 274 (JP); SHARYOU, Masaki, Takamidai Telass House, Chiba-ken 270 (JP)
(74) Representative: Knudsen, Sten Lottrup
(86) International application number: DK9200137
(87) International publication number: WO9219808

(56) References cited:
- EP-A- 0 374 700
- GB-A- 1 189 604
- CHEMICAL ABSTRACTS, Volume 107, No. 25, 21 December 1987, (Columbus, Ohio, US),j: "Hydrolysis of triglyceride in two-phase system using immobilized lipase",see page 630, Abstract 234795t, & Sanop Misaengmul Hakhoechi 1987, 15(2), 122-128.

## Description

### TECHNICAL FIELD

This invention relates to a process for hydrolysis of water-insoluble ester in the presence of a lipase, particularly to such a process for hydrolysis of pitch (resin) in pulp, and to a method of increasing the rate of hydrolysis of water-insoluble ester in the presence of a lipase by incorporation of a polyelectrolyte.

### BACKGROUND ART

It is known that lipases can be used with advantage for efficient hydrolysis of water-insoluble esters, particularly triglycerides (e.g. JP-A 51-080305, JP-A 58-126794, JP-A 59-210893, GB-A 2,176,480, WO 88/02775).

It is also known that some types of pulp made from wood have a high pitch content, e.g. various types of mechanical pulp. This can cause so-called pitch troubles in papermaking such as paper contamination or paper breaks. Pitch contains considerable amounts of triglycerides, more commonly known as fats, and other esters.

It is the object of this invention to provide an improved process for ester hydrolysis, applicable to hydrolysis of resin esters.

### STATEMENT OF THE INVENTION

We have found that, surprisingly, addition of a water-soluble polyelectrolyte (i.e. an anionic or cationic polymer) significantly increases the hydrolysis rate of esters in the presence of lipases.

Various metal cations have been reported to affect lipase activity, and cationic surfactant has been reported inhibit lipase activity (Nishio et al., *Agric. Biol. Chem.*, 51 (1), 181-186, 1987; C.E. Ibrahim et al., *Agric. Biol*. *Chem.*, 51 (1), 37-45, 1987). The effect of polyelectrolytes on lipase activity has not been described.

Accordingly, the invention provides a process for hydrolysis of water-insoluble ester in the presence of a lipase, characterized by the presence of a water-soluble polyelectrolyte. The invention also provides a method of increasing the rate of hydrolysis of water-insoluble ester in the presence of a lipase by incorporation of a water-soluble polyelectrolyte.

### DETAILED DESCRIPTION OF THE INVENTION

### Polyelectrolyte

The polyelectrolyte used in the invention may be any water-soluble polymer that contains functional groups which ionize in water. It may be cationic or anionic. A group of preferred anionics is anionic polyacrylamide, e.g. a copolymer of acrylamide and acrylate (such as sodium acrylate).

Some preferred cationic polymers are those contaning tertiary or quaternary amine groups. An example is cationic starch having diethylamino-ethyl groups or 2-hydroxy,2-(trimethylamino-methyl)ethyl groups attached to the hydroxyl group in the 6-position of the repeating glucose unit of the starch molecule.

Another example is cationic polyacrylamide, e.g. a copolymer of acrylamide with N-(dimethyl-amino-methyl)-acrylamide, dimethyl-amino-ethyl methacrylate or trimethyl-amino-ethyl methacrylate. A further example is cationic polyamine such as quaternary polyamine and polyethyleneimine.

Use of the above-mentioned polyelectrolytes is particularly advantageous in papermaking where these polymers may simultaneously act as flocculants or retention aids.

The amount of polyelectrolyte is preferably 2-1000 ppm, preferably 10-200 ppm in the reaction mixture, or 0.1-10 kg/ton of dry matter, particularly 0.3-3 kg/t.

### Lipase

For reasons of economy, microbial lipases are preferred. Examples of suitable enzymes are lipases derived from strains of *Pseudomonas* (especially *Ps. cepacia*, *Ps. fluorescens*, *Ps. fragi* and *Ps. stutzeri*), *Candida* (especially *C. antarctica* (e.g. lipase A or B, see WO 88/02775) and *C. cylindracea*), *Humicola* (especially *H. brevispora*, *H. lanuginosa*, *H. brevis var. thermoidea* and *H. insolens*), *Chromobacterium* (especially *C. viscosum*) and *Aspergillus* (especially *A. niger*).

The amount of lipase will typically correspond to a lipase activity of 1,000-100,000 LU/kg dry matter or 50-5,000 LU/litre (LU = Lipase Unit, defined in WO 89/04361).

### Ester hydrolysis process

Typical process conditions are pH 3-7.5, particularly 4-7, a temperature from ambient to 80°C, particularly 30-60°C, and reaction times of 0.5-3 hours.

The process of the invention can be used for any lipase-catalyzed hydrolysis of water-insoluble esters, particularly triglycerides.

Thus, the process of the invention may be used for fat hydrolysis in the production of fatty acids, glycerides and/or glycerol from fat or oil. The ester may be a liquid at ambient temperature, such as soy bean oil and many other oils, or it may be a high melting fat, such as beef tallow.

### Hydrolysis of resin esters

The process of the invention is particularly applicable to the hydrolysis of resin esters during a pulping or paper-making process, e.g. to avoid pitch troubles such as paper contamination, paper breaks or contamination of process equipment.

The process of the invention may be applied to any pitch-containing pulp, especially to pulps with a considerable content of triglycerides and other esters from pitch. Examples are pups produced by mechanical pulping, alone or combined with a gentle chemical treatment, such as GW (Ground Wood), TMP (Thermo Mechanical Pulp) and CTMP (Chemical Thermo Mechanical Pulp).

Hydrolysis of esters in pitch according to the invention can be done in the pulping or stock preparation section, where addition of polyelectrolytes is particularly advantageous since it can also act as a retention or flocculation aid. The pulp typically has a consistency of 0.2-5% dry substance.

### EXAMPLES

### EXAMPLE 1

Red pine (Pinus radiata) ground wood pulp was treated with *Humicola* lipase in the presence of various polyelectrolytes. After the reaction the degree of triglyceride hydrolysis was determined by quantitative TLC using latroscan™.

Conditions were: 4% pulp slurry, pH 4.5, temperature 40°C, agitation 300 rpm. The dosage of polyelectrolyte and enzyme is given below as ppm/DS. The results are expressed as the amount of triglycerides relative to a control with lipase alone.

| Polyelectrolyte | Dosage of poly. (ppm/DS) | Dosage of Lipase (ppm/DS) | Relative Amount of Triglycerides (*) (%) |
|---|---|---|---|
| None (control) | 0 | 1000 | 100 |
| Anionic, High Molecular Polyacrylamidecopolymer | 1000 | 1000 | 79 |
| Cationic, High Molecular Polyacrylamidecopolymer | 1000 | 1000 | 67 |
| Strongly Cationic, High Molecular Polyacrylamidecopolymer | 1000 | 1000 | 64 |
| Quaternary Polyamine | 1000 | 1000 | 67 |
| Cationic Polymer | 1000 | 1000 | 71 |

| | | | |
|---|---|---|---|
| (*): Determined by quantitative TLC; Iatroscan Method. | | | |

It is seen that all the anionic and cationic polymers tested increased the hydrolysis of triglyceride.

### EXAMPLE 2

To verify the effect of polyelectrolytes on lipase activity another experiment was done, using two different cationic polymers. Conditions were: 4% pulp slurry, pH 4.5, temperature 40°C, 2 hours reaction time, agitation 300 rpm. Dosage of polyelectrolytes and enzyme are given below as ppm/DS. The results are expressed as amount of triglycerides relative to the amount without lipase.

| Dosage (ppm/DS) of | | Dosage (ppm/DS) of Lipase | Relative amount Triglycerides (%) |
|---|---|---|---|
| Cationic Polymer | Quarternary Polyamine | | |
| 0 | 0 | 0 | 100 |
| 0 | 0 | 1000 | 45 |
| 1000 | 0 | 1000 | 36 |
| 1000 | 0 | 0 | 100 |
| 0 | 1000 | 1000 | 32 |
| 0 | 1000 | 0 | 100 |

| | | | |
|---|---|---|---|
| (*): Determined by quantitative TLC; Iatroscan Method. | | | |

## Claims

1. A process for hydrolysis of water-insoluble ester in the presence of a lipase, characterized by the presence of a water-soluble polyelectrolyte.

2. A process according to Claim 1, wherein the polyelectrolyte has cationic groups, preferably tertiary or quaternary amine groups.

3. A process according to Claim 2, wherein the cationic polyelectrolyte is cationic starch or a copolymer of acrylamide with N-(dimethyl-amino-methyl)acrylamide, dimethyl-amino-ethyl methacrylate or trimethyl-amino-ethyl methacrylate.

4. A process according to Claim 1, wherein the polyelectrolyte has anionic groups, preferably carboxyl groups.

5. A process according to Claim 4, wherein the anionic polyelectrolyte is a copolymer of acrylamide and acrylate.

6. A process according to any preceding claim, wherein the polyelectrolyte is present in an amount of 2-1000 ppm, preferably 10-200 ppm.

7. A process according to any preceding claim, wherein the pH is in the range 3-7.5, preferably 4-7.

8. A process according to any preceding claim in the presence of a microbial lipase, preferably derived from a strain of *Candida*, *Pseudomonas*, *Humicola*, *Chromobacterium* or *Aspergillus*, and preferably at a lipase activity of 1-100 KLU/kg of dry matter.

9. A process according to any preceding claim for hydrolysis of esters in pitch during a pulping or paper-making process.

10. A process according to the preceding claim, whereby the cellulase activity is below 1000 EGU/kg.

11. A method of increasing the rate of hydrolysis of water-insoluble ester in the presence of a lipase by incorporation of a water-soluble polyelectrolyte.

## Patentansprüche

1. Ein Verfahren zur Hydrolyse von wasserunlöslichem Ester in der Gegenwart einer Lipase, gekennzeichnet durch die Gegenwart eines wasserlöslichen Polyelektrolyten.

2. Ein Verfahren nach Anspruch 1, wobei der Polyelektrolyt kationische Gruppen besitzt, vorzugsweise tertiäre oder quaternäre Amingruppen.

3. Ein Verfahren nach Anspruch 2, wobei der kationische Polyelektrolyt kationische Stärke oder ein Copolymer von Acrylamid mit N-(Dimethylaminomethyl)acrylamid, Dimethylaminoethylmethacrylat oder Trimethylaminoethylmethacrylat ist.

4. Ein Verfahren nach Anspruch 1, wobei der Polyelektrolyt anionische Gruppen besitzt, vorzugsweise Carboxylgruppen.

5. Ein Verfahren nach Anspruch 4, wobei der anionische Polyelektrolyt ein Copolymer von Acrylamid und Acrylat ist.

6. Ein Verfahren nach einem vorangehenden Anspruch, wobei der Polyelektrolyt in einer Menge von 2-1000 ppm, vorzugsweise 10-200 ppm vorhanden ist.

7. Ein Verfahren nach einem vorangehenden Anspruch, wobei der pH im Bereich 3-7,5 liegt, vorzugsweise 4-7.

8. Ein Verfahren nach einem vorangehenden Anspruch in der Gegenwart einer mikrobiellen Lipase, vorzugsweise gewonnen aus einem Stamm von Candida, Pseudomonas, Humicola, Chromobacterium oder Aspergillus, und vorzugsweise bei einer Lipaseaktivität von 1-100 KLU/kg Trockensubstanz.

9. Ein Verfahren nach einem vorangehenden Anspruch zur Hydrolyse von Estern in Harz während eines Pulpeherstellungs- oder Papierherstellungsverfahrens.

10. Ein Verfahren nach dem vorangehenden Anspruch, wobei die Cellulaseaktivität unterhalb von 1000 EGU/kg liegt.

11. Ein Verfahren zur Erhöhung der Geschwindigkeit der Hydrolyse von wasserunlöslichem Ester in der Gegenwart einer Lipase durch Einbeziehung eines wasserlöslichen Polyelektrolyten.

## Revendications

1. Procédé pour l'hydrolyse d'ester insoluble dans l'eau en présence d'une lipase, caractérisé par la présence d'un polyélectrolyte hydrosoluble.

2. Procédé selon la revendication 1, dans lequel le polyélectrolyte possède des groupes cationiques, de préférence des groupes amine, tertiaire ou quaternaire.

3. Procédé selon la revendication 2, dans lequel le polyélectrolyte cationique est de l'amidon cationique ou un copolymère d'acrylamide avec N-(diméthyl-amino-méthyl)acrylamide, diméthyl-amino-éthyle méthacrylate ou triméthyl-amino-éthylméthacrylate.

4. Procédé selon la revendication 1, dans lequel le polyélectrolyte possède des groupes anioniques, de préférence des groupes carboxyle.

5. Procédé selon la revendication 4, dans lequel le polyélectrolyte anionique est un copolymère d'acrylamide et d'acrylate.

6. Procédé selon l'une quelconque des revendications précédentes, dans lequel le polyélectrolyte est présent dans une quantité de 2-1000 ppm, de préférence 10-200 ppm.

7. Procédé selon l'une quelconque des revendications précédentes, dans lequel le pH se situe dans la plage 3-7,5, de préférence 4-7.

8. Procédé selon l'une quelconque des revendications précédentes en présence d'une lipase microbienne, de préférence dérivée de la souche *Candida*, *Pseudomonas*, *Humicola*, *Chromobacterium* ou *Aspergillus*, et de préférence à une activité lipase de 1-100 KUL/kg de matière sèche.

9. Procédé selon l'une quelconque des revendications précédentes pour l'hydrolyse d'ester dans une résine pendant un procédé de réduction en pâte ou de fabrication du papier.

10. Procédé selon la revendication précédente, dans lequel l'activité de la cellulase est inférieure à 1000 EGU/kg.

11. Procédé pour augmenter la vitesse d'hydrolyse d'ester insoluble dans l'eau en présence d'une lipase en incorporant un polyélectrolyte hydrosoluble.
